# EUROPEAN PATENT APPLICATION

(11) **EP 3 650 051 A2**
(43) Date of publication of application: **13.05.2020**
(21) Application number: 19204234.9
(22) Date of filing: 21.10.2019
(51) Int. Cl.: A61L 9/12

(54) **PASSIVE FRAGRANCE DISPENSER SYSTEM**

(30) Priority: 21.10.2018 US 201862748490 P; 16.10.2019 US 201916654581
(71) Applicant: San Jamar, Inc., Elkhorn, WI 53121 (US)
(72) Inventor: ZIEBART, Bernie, Pewaukee, WI Wisconsin 53972 (US); SCHOON, Jodie, East Troy, WI Wisconsin 53120 (US)
(74) Representative: Barker Brettell LLP

(57) **Abstract**

An air treatment media dispenser assembly (50) that passively delivers an air treatment media to an environment. The dispenser assembly includes a carriage (52) that is constructed to removably cooperate with a replaceable container (54). The container slidably cooperates with the carriage in a manner wherein the container is secured to the carriage without perforation of the container and, when axially translated relative to one another, perforates a seal (108) of the container to allow egress of the contents of the container to the carriage and subsequent passive communication of the media to an environment.

## Description

### CROSS REFERENCE TO RELATED APPLICATIONS

This application claims priority to U.S. Provisional Patent Application Ser. No. 62/748,490 filed on October 21, 2018 titled "Passive Fragrance Dispenser System" and the disclosure of which is expressly incorporated herein.

### BACKGROUND OF THE INVENTION

The present invention relates generally to fragrance dispensing systems and, more particularly, to a fragrance dispenser assembly that is constructed to passively dispense fragrance via evaporation of an air care media via environmental flow streams through a housing of the dispenser assembly. The present invention is further directed to a replaceable media container that is opened to expose the media to the treatment environment via axial cooperation of the container with a container support, carriage, or housing of the dispenser assembly.

Fragrance dispensers are well known and have applicability across a number of environments including rest rooms and the like. Many such dispensers include heating elements, blowers, or fans associated with improving the dispersal of the air treatment media to the environment being treated. Such assemblies suffer from various drawbacks including the requirement of grid or battery power associated with operation of the heating and/or extraneous air flow creating elements. Such requirements increase manufacturing costs as well as the cost associated with implementation and maintaining operability of such systems. When not properly maintained, such approaches can also result in overtreatment of the operating environments and/or extended periods of non-treatment of the operating environment when the dispensing assembly achieves an "empty" condition shortly after being serviced but well before a subsequent service interval.

Still further, other systems can require convoluted and complicated manipulation of media containers and/or components thereof relative to the underlying dispenser assemblies to prevent undesired spillage of the media contents and/or to achieve the intended operation of the dispenser assembly. Some such system require introduction of the treatment media to a dispenser supported reservoir in a manner that can lead to spillage or spoilage of the media prior to use or consumption via in an evaporative manner. Still others require replenishment of the media from bulk containers that commonly require storage and/or repeated transport of previously opened bulk containers. Such approaches can result in spillage of the treatment media at locations remote for the dispensing assembly and the intended operating environment and/or undesired evaporation of the media from the bulk container when stowed. Such approaches can further result in incidents of multiple bulk containers being maintained in an unsealed state when multiple persons are charged with replenishing or refilling dispensers deployed at various discrete locations. Such concerns can exacerbate the waste or spoilage of the treatment media prior to use of the same.

Therefore, the need exists for an air care treatment dispensing system that can be economically manufactured, deployed, and maintained and which includes limited consumable or disposable components associated with continued intended air conditioning operation of the device. There is therefore a further need for an air care dispenser assembly that is simple and convenient to operate as well as service.

### SUMMARY OF THE INVENTION

The present invention discloses an air care treatment dispensing system that solves one or more of the shortcomings disclosed above. The media dispensing system according to one aspect of the present invention includes a media container that axially cooperates with a carriage to effectuate loading and puncturing the sealing feature associated with maintaining the sealed condition of the media until such time as use of the same is desired. It is appreciated that the containers may be one or more of refillable and/or recyclable.

Another aspect of the application that is usable or combinable with the above aspects, objects, and features discloses a passive fragrance dispenser assembly having a carriage that extends along a longitudinal axis and a cartridge that defines a cavity configured to contain an amount of an air treatment media and which slideably cooperates with the carriage. A barb is defined by the carriage and oriented to pierce a seal associated with the cartridge to allow egress of the air treatment media from the cartridge in response to axial translation of the cartridge relative to the carriage and the barb.

A further aspect of the present application that is usable or combinable with one or more of the above features, aspects, and objects discloses an air treatment media dispenser assembly having a container that is defined by a body and a blind opening. A seal is disposed over the opening and a carriage is constructed to slideably receive the container when the seal is oriented in a downward facing direction and secure the container relative to the carriage without interference or interaction with the seal. A projection that is defined by the carriage is configured to engage and pierce the seal to fluidly connect a volume defined by the container to atmosphere during axial translation of the container toward the carriage in an axial direction.

Another aspect of the application that is combinable or usable with one or more of the above aspects, features, and objects discloses a method of dispensing an air treatment media to an environment. The method includes associating a sealed cartridge that contains an air treatment media with a carriage such that translation of the sealed cartridge in a downward non-rotational axial direction relative to the carriage punctures a seal supported by cartridge so that the air treatment media can populate a basin defined by the carriage.

These and other aspects, features, and advantages of the present invention will become apparent from the detailed description, claims, and accompanying drawings.

### DESCRIPTION OF THE DRAWINGS

A clear conception of the advantages and features constituting the present invention, and of the construction and operation of typical mechanisms provided with the present invention, will become more readily apparent by referring to the exemplary, and therefore non-limiting, embodiments illustrated in the drawings accompanying and forming a part of this specification, wherein like reference numerals designate the same elements in the several views, and in which:
**FIG. 1** is a perspective view of a fragrance media dispenser system or assembly having a media cartridge and cartridge support assembly according to the present invention;
**FIG. 2** is a bottom plan view of the fragrance media dispenser assembly shown in FIG. 1;
**FIG. 3** is a perspective view of the fragrance media dispenser assembly shown a FIG. 1 with a cover portion and a replaceable media container and container carriage exploded therefrom;
**FIG. 4** is a perspective view of a media cartridge or container and cartridge support or carriage assembly removed from the fragrance media dispenser assembly shown in FIG. 1;
**FIG. 5** is a side elevation view of the media container and cartridge support assembly shown in FIG. 4;
**FIG. 6** is a top plan view of the media cartridge and cartridge support assembly shown in FIG. 4;
**FIG. 7** is an exploded view of the media cartridge or container, an optional sleeve, and the cartridge support assembly shown in FIG. 4;
**FIG. 8** is a longitudinal cross section view of the media cartridge and cartridge support assembly shown in FIG. 4 taken along line 8-8 shown in

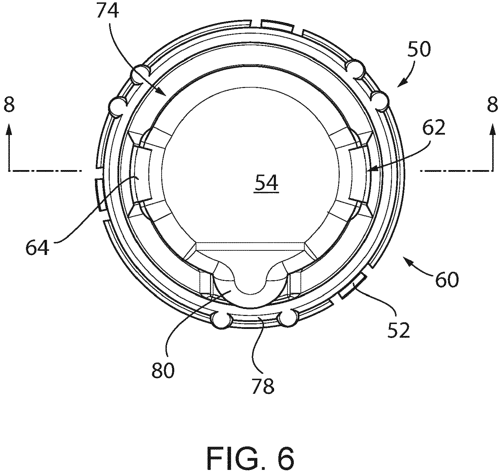

**FIG. 6** and associated with a first or sealed orientation of the media container relative to the carriage;

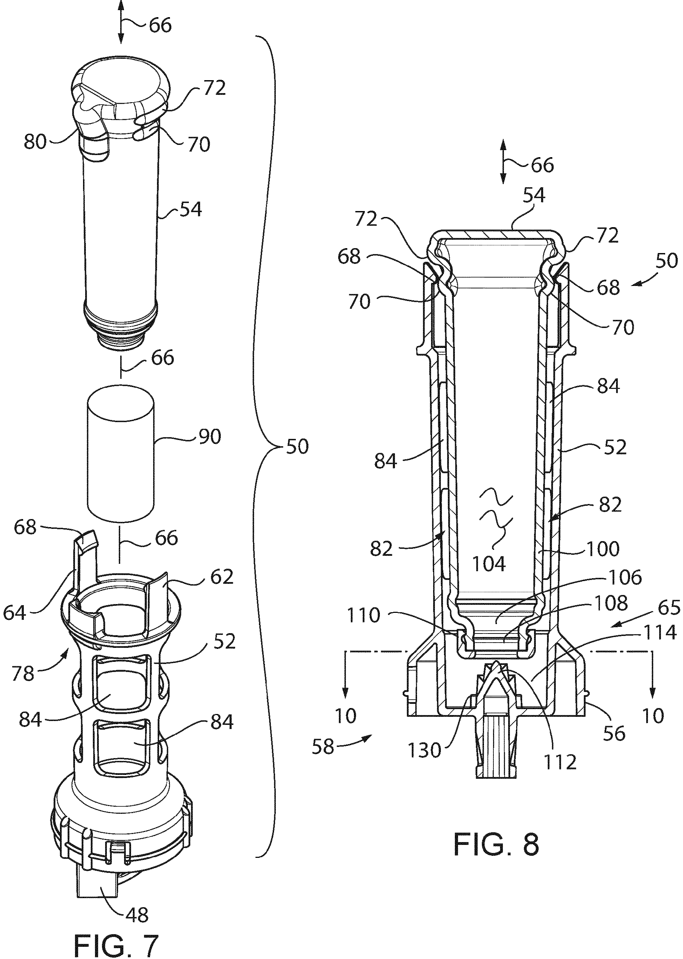

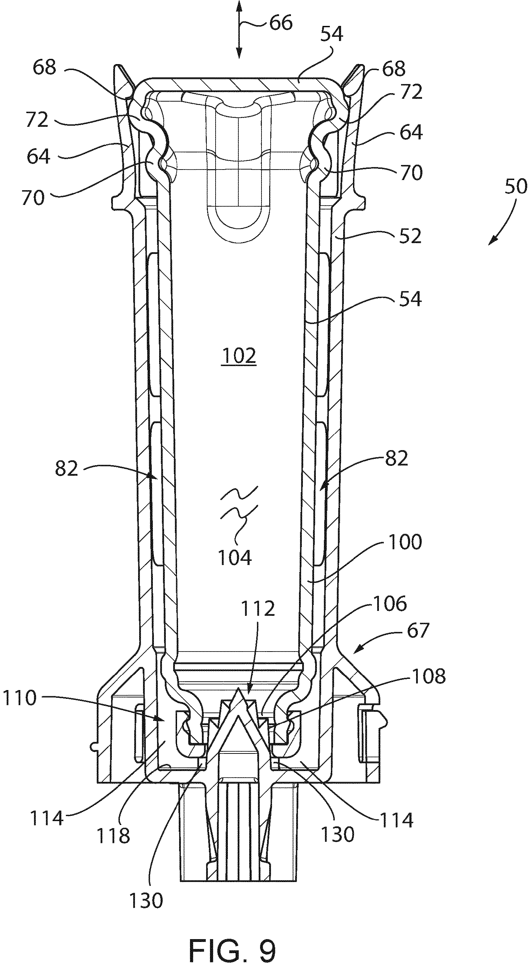

**FIG. 9** is a view similar to FIG. 8 and shows the media container in a second orientation relative to the cartridge support assembly wherein the media associated with the container is exposed to the operating environment;

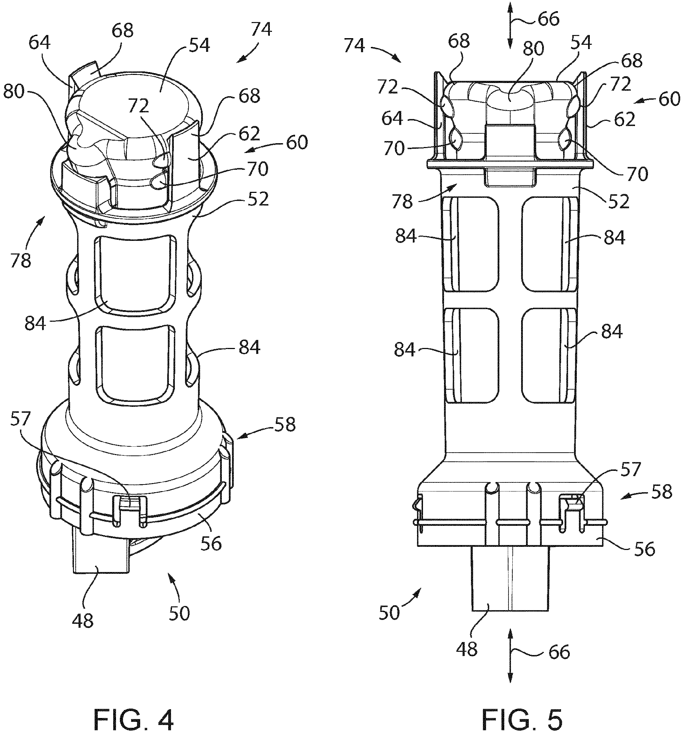

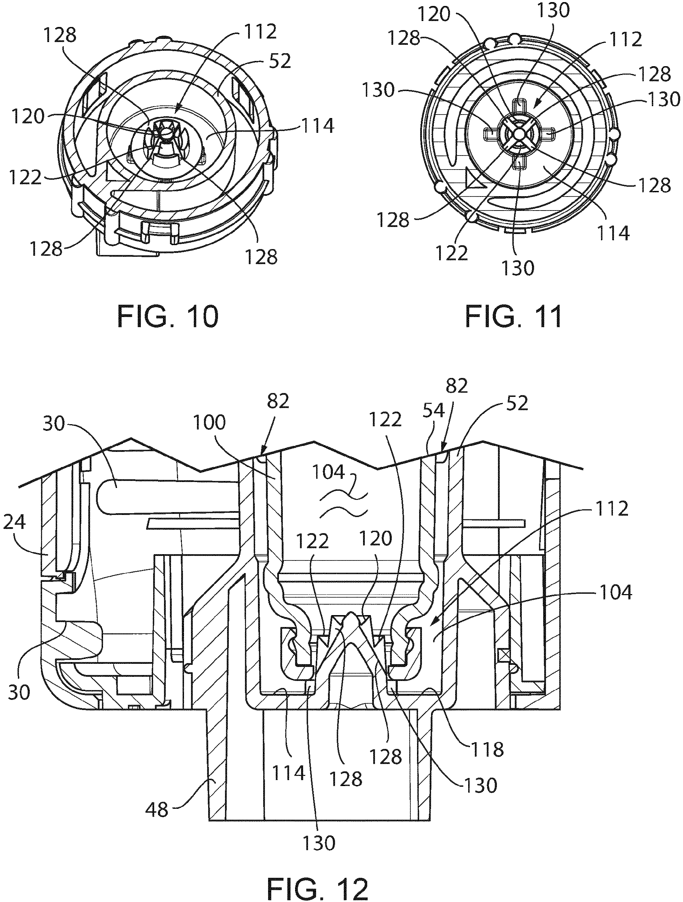

**FIG. 10** is a cross section perspective view of the cartridge support assembly shown in FIG. 4 taken along line 10-10 shown in FIG. 8 and shows a media basin defined thereby;
**FIG. 11** is a top plan view of the media basin shown in FIG. 10; and

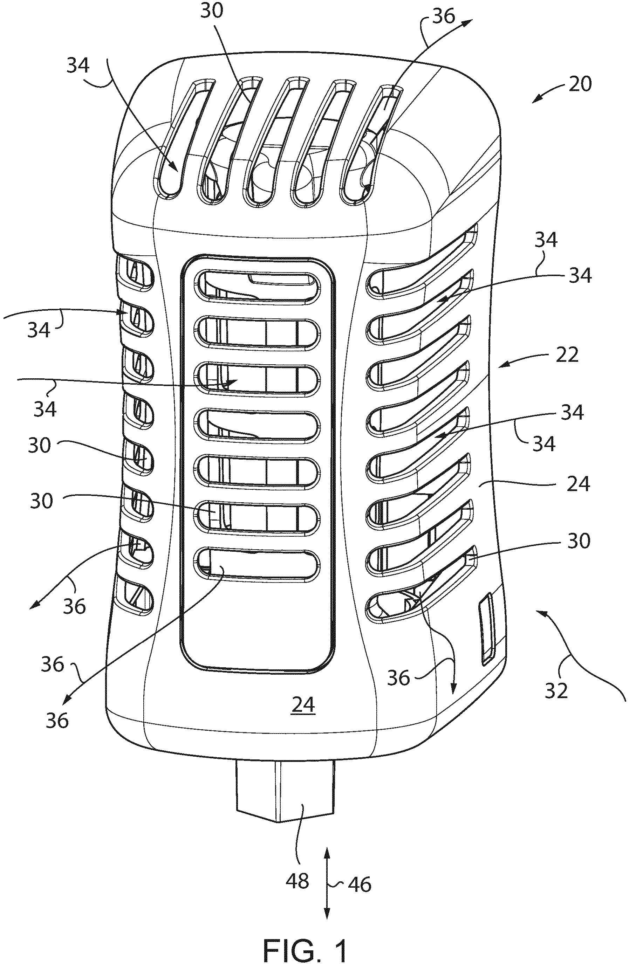

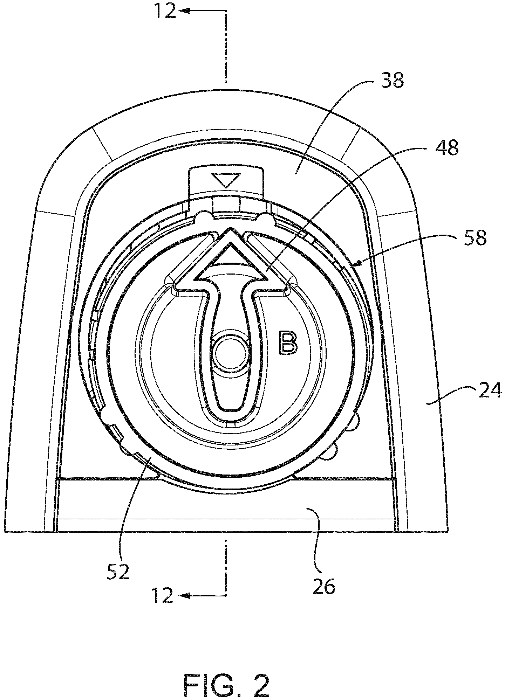

**FIG. 12** is a detailed elevation cross-section view of the media dispenser assembly shown in FIG. 1 taken along line 12-12 shown in FIG. 2.

### DETAILED DESCRIPTION OF THE PREFERRED EMBODIMENTS

In describing the embodiments of the invention which are illustrated in the drawings, specific terminology will be resorted to for the sake of clarity. However, it is not intended that the invention be limited to the specific terms so selected and it is to be understood that each specific term includes all technical equivalents which operate in a similar manner to accomplish a similar purpose. The various features and advantageous details of the subject matter disclosed herein are explained more fully with reference to the non-limiting embodiments described in detail in the following description.

Illustrative components of a passive fragrance media delivery assembly or system or media delivery system 20 in accordance with various aspects of the present invention are shown in Fig. 1 through Fig. 12. Referring to Figs. 1-3, media delivery system, passive fragrance delivery system or assembly or simply delivery system 20 preferably includes an enclosure or housing 22 defined by a cover 24 and a base 26 that is disposed therebehind. Although cover 24 and base 26 preferably movably cooperate with one another to expose a volume 28 contained thereby and to facilitate mounting of housing 22, it is appreciated that housing 22 could be provided in a number of shapes and sizes and need not be constructed to provide a movable connection between cover 24 and base 26. That is, it is appreciated that housing 22 could be provided in a generally unitary construction.

Housing 22 includes a support 38 that extends between cover 24 and base 26 proximate the lower end of housing 22. In a preferred aspect, support 38 snap-fittingly cooperates with base 26 when oriented to extend between cover 24 and base 26. As disclosed further below, support 38 defines a passage of bore 40 that is constructed to removeably cooperate with a dispenser assembly 50. As disclosed further below, in a preferred embodiment, dispenser assembly 50 snap-fittingly cooperates with support 38 when the dispenser assembly 50 is associated with housing 22 although it is appreciated that other interfering engagements, such as rotatable or the like, could provide the selectively securable but periodically removable interaction between dispenser assembly 50 and housing 22.

Regardless of the specific construction methodology employed, one or more of cover 24 and base 26 include a plurality of vents 30 that allow passage of ambient airflow, indicated by arrow 32 into, indicated by arrows 34, and out of, indicated by arrows 36, housing 22. Passage of ambient airflows 32 into 34 and out of 36 housing 22 allows introduction of an air treatment media as disclosed further below into airstream 32 when delivery system 20 is deployed and configured for treatment of the airflow. As disclosed above, it is appreciated that the housing 22 can be provided in any number of shapes, sizes, and appearances other than that which is shown. In a preferred embodiment, system 20 includes no power consuming elements within housing 22 and housing 22 includes a plurality of vents 30 constructed to facilitate communication of the ambient air flows into and out of housing 22.

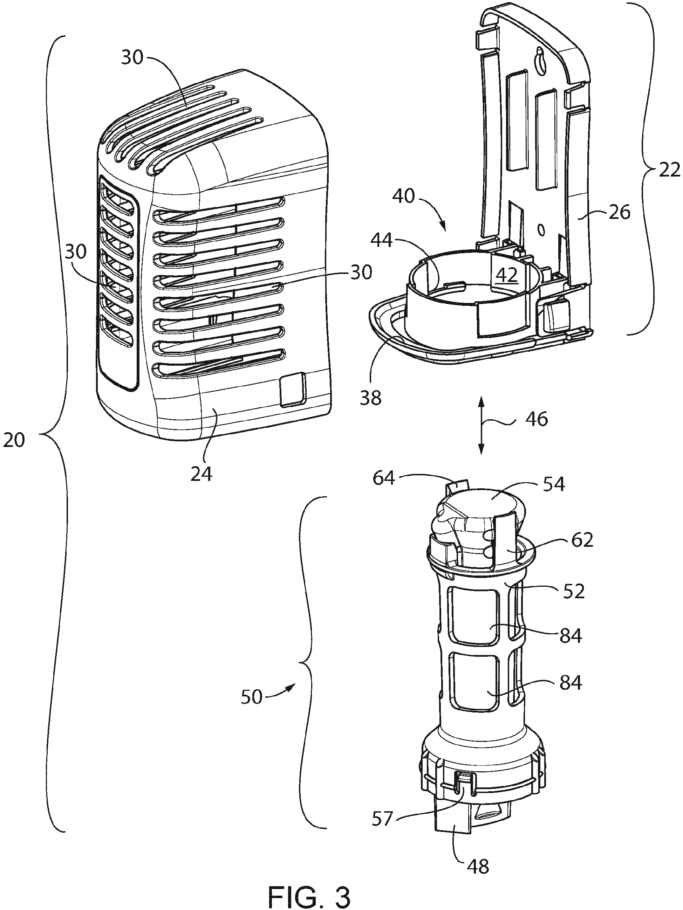

Referring to Figs. 3-5, passive air treatment delivery system 20 includes a dispenser assembly 50 that is constructed to be disposed in housing 22 and to be removeable therefrom. Dispenser assembly 50 includes a base, support, or carriage 52 that is constructed to slidably cooperate with a container 54 that contains a volume of an air treatment media as disclosed further below. It is appreciated that the air treatment media may be provided in any number of compositions and that such compositions can be configured to provide a desired fragrance and/or air flow sanitation features.

As shown in Fig. 3, an interior contour 42 associated with bore 40 of support 38 of housing 22 includes one or more detents 44 that are constructed and oriented to cooperate with respective projections or tangs 57 associated with a lower end 58 of carriage 52 when carriage 52 is associated with housing 22 via bore 40. It should be understood that dispenser assembly 50 slideably cooperates with housing 22 via interaction with bore 40 in longitudinal directions 46 during placement and extraction or removal of dispenser assembly 50 therefrom. As disclosed further below, container 54 of dispenser assembly 50 longitudinally slideably cooperates with carriage 52 thereof to effectuate replacement of container 54 when required. Dispenser assembly 50 includes a handle 48 that is accessible from outside housing 22 to facilitate the placement and removal of dispenser assembly 50 from housing 22. Handle 48 also provides an indication as to the rotational orientation of carriage 52 relative to housing 22 to provide the desired slideable and snap-fitting interaction of carriage 52 with bore 40 of support 38. As shown, handle 48 has a generally arrow shape wherein when the tip of the arrow faces in a forward facing direction relative to a housing 22 hung in a vertical orientation, detents 44 and tangs 57 are rotationally aligned relative to one another to ensure the desired snap-fitting cooperation when dispenser assembly 50 is slid into housing 22 via bore 40. Although provided in a convenient and readily operable manner from positions external to housing 22, it is appreciated that housing 22 could be provided in various configurations and constructions to provide controlled selective access to dispenser assembly 50 for replenishment or refilling of the same. For instance, it is appreciated that providing cover 24 in a construction wherein cover 24 movable or removeably cooperates with base 26 and/or support 38 would also provide selective controlled access to dispenser assembly 50 albeit in the manner that is less convenient for service personnel or the like.

As shown in Figs. 3-5, carriage 52 is generally defined by an elongate body 56 that extends between a lower end 58 and an upper end 60. One or more tangs 62, 64 extend in an upward longitudinal direction, indicated by arrow 66, relative to body 56 of carriage 52. Each of tangs 62, 64 includes a barb 68 that extends in an inward radial direction relative to longitudinal axis 66 from distal ends of respective tangs 62, 64. Barbs 68 preferably snap-fittingly cooperate with a respective groove defined by respective projections 70, 72 associated with an upper distal end 74 of container 54. Preferably, tangs 62, 64 provide one or more of a tactile and/or audible indication as to a relative longitudinal position of container 54 relative to carriage 52.

Tangs 62, 64 cooperate with container 54 in a manner wherein, in a first orientation 65 (Fig. 8), container 54 is secured to carriage 52 but the contents thereof are not fluidly exposed, and a second orientation 67 (Fig. 9) wherein container 54 is secured to carriage 52 and fluid communication therebetween is facilitated. The first orientation 65 allows dispenser assembly 50 to be provided in a plug-and-play methodology wherein dispenser assembly 50 can be manufactured, stored, and shipped to consumers in a non-vented format preventing consumption, waste, or spoilage of the treatment media until desired. Such an understanding is disclosed further below with respect to Figs. 8-12. Such considerations allow dispenser assembly 50 to be provided to consumers in a form factor wherein placement of the dispenser assembly 50 relative to housing 22, and subsequent longitudinal translation of container 54 relative to carriage 52, renders the dispenser assembly operational for dispensing of the media. As disclosed further below, container 54 is supported by or otherwise secured to carriage 52 in a manner wherein the contents of the container 54 are sealingly contained thereby until desired to be passively dispensed.

Still referring to Figs. 3-5, upper end 60 of carriage 52 includes an index structure 78 that is constructed to slidably cooperate with another index structure or projection 80 that extends in an outward radial direction relative to upper distal end 74 of container 54. Container 54 is axially slideable and non-rotationally cooperates with carriage 52 so as to be axially translatable relative thereto in a longitudinal direction aligned with axis 66.

Carriage 52 includes one or more cutouts or openings 84 that extend in a radial direction relative to axis 66 through a body 76 of carriage 52 and allows exposure of an optional sleeve 90 (Fig. 7) that is disposed between carriage 52 and container 54 as disclosed further below. To improve the visibility of the surrounding structures, it should be understood that sleeve 90 is only shown in Fig. 7 and is constructed to be disposed in the radially and longitudinally extending gap 82 (Fig. 8) between the interior facing surface of carriage 52 and the radially exterior facing surface of container 54 and without interfering with the interaction between the respective upper and lower ends of the carriage 52 and discrete containers 54 associated therewith.

As disclosed further below, sleeve 90 is preferably constructed of materials suitable to effectuate capillary activity associated with passively dispensing treatment media dispensed from container 54 to ambient airflows 32, 34, 36 that travel thereacross when dispenser assembly 50 is deployed. That is, sleeve 90 is conducive to capillary transport of the treatment media as well as evaporative surrender of the media to the ambient air flows thereacross. It is further appreciated that altering the material of sleeve 90 can alter both the capillary and evaporative response of the same. It is further appreciated that, for those instances wherein less evaporation of the media is desired, sleeve 90 may be omitted such that only the portion of the reservoir or basin defined by carriage 52 as disclosed further below may provide the flow and surface area associated with evaporation of the treatment media.

Referring to Figs. 4-6, head or upper portion or distal end 74 of container 54 and upper end 60 of carriage 52 are shaped to provide a desired relative rotational orientation of container 54 relative to carriage 52 to facilitate the desired axial translation of container 54 relative to sleeve 90 and carriage 52. As shown in Fig. 5, rotational alignment of projection 80 defined by container 54 with index structure 78 defined by carriage 52 aligns tangs 62, 64 with respective projections 70, 72 associated with the generally opposite radial sides of container 54 such that container 54 is axially translatable relative to sleeve 90 and carriage 52 as described further below. It should further be appreciated that upper end 74 of container 54 has a slightly downward directed frusto-conical shape to allow a user to conveniently grasp container 54 during removal and/or replacement of container 54 from carriage 52 and/or removal of container 54 and carriage 52 from dispenser housing 22 as disclosed further below during replacement or replenishment of dispenser assembly 50. As disclosed above and as shown in Fig. 2, lower end 58 of carriage 52 includes handle 48 and is shaped to slideably cooperate with bore 40 of support 38 to indicate the desired rotational orientation of dispenser assembly 50 relative to housing 22 during placement and/or removal of dispenser assembly 50 from housing 22. Such considerations allow service personnel to expeditiously remove, replenish, replace, and/or otherwise service dispenser assemblies 50 having spent or consumed media containers 54.

Referring to Figs. 8-12, container 54 is constructed to cooperate with carriage 52 in respective first and second positions or orientations 65, 67 wherein container 54 is secured thereto but is axially translatable relative to carriage 52 to facilitate puncturing of a seal associated therewith. Referring to Fig. 8, container 54 is defined by a body 100 that generally surrounds a cavity 102, that when full, is populated by a liquid air treatment media 104 contained therein. Container 54 defines an opening 106 having a seal 108 disposed there over. As is customary, seal 108 is commonly formed of a plastic and/or cellophane material and/or foil reinforced cellophane or plastic material. Such seals are commonly plastically deformable such that the same must commonly be removed before the contents of such sealed containers can be introduced to the dispenser assembly. Such an approach is rendered unusable for use in the present dispenser assembly in as much as the open end of the container must be oriented in a downward facing direction during loading of the dispenser assembly for use. Such approaches also frequently result in spillage of the media during loading operations. Other puncturing operations commonly require multiple direction, such as axial and rotational translation, of the container to overcome the plastic deformation of such seals to achieve an "open" or perforated condition thereof. Unlike other systems, dispenser assembly 50 does not require such multiple direction manipulations by the construction and operation of dispenser assembly 50 as disclosed further below.

Dispenser assembly 50 is constructed to achieve perforation of seal 108 in a manner that allows egress of media 104 from container 54 in response to solely axial translation of container 54 relative to carriage 52. Such an approach simplifies deployment of dispenser assembly 50 in achieving the in-use configuration of dispenser assembly 50. When assembled, but not fluidly connected, tangs 62, 64 defined by carriage 52 cooperate with projections 70 of container 54 such that a lower end 110 of container 54 associated with seal 108 is disposed in close but not interfering interaction with a piercing structure or barb 112 defined by carriage 52. Downward longitudinal displacement of container 54 relative to carriage 52 allows barb 112 to penetrate or otherwise perforate seal 108 associated with container 54 such that the contents thereof can populate a basin 114 defined by carriage 52.

During downward longitudinal translation of container 54 relative to carriage 52, outward radial deflection of tangs 62, 64 allow barbs 68 to translate over projections 72 and maintain a second orientation of container 54 relative to carriage 52 wherein barb 112 pierces seal 108. Piercing of seal 108 allows media 104 previously sealingly contained in container 54 to occupy basin 114 and, when employed, exposes sleeve 90 to media 104 via basin 114 such that capillary action allows media 104 to translate and populate sleeve 90 to those areas generally overlying openings 84 defined by carriage 52. The cooperation of tangs 62, 64 and barbs 68 with projections 72 of container 54 maintain a secured and gravitationally sealed orientation of basin 114 such that media 104 is dispensed in a passive manner via evaporative interaction of the ambient air flows 32, 34, 36 with the exposed areas of sleeve 90 and/or the fluid/gas interface layer associated with the media 104 disposed in basin 114.

Referring to Figs. 9-12, barb 112 is constructed to facilitate piercing of seal 108 in response to axial translation of container 54 relative to carriage 52. Barb 112 extends in an upward axial direction 116 relative to a bottom 118 of basin 114. Barb 112 includes a number of projections 120, 122 that are oriented generally concentrically and at varying elevations relative to bottom 118 of basin 114. Projections 120, 122 of barb 112 are separated from one another by one or more channels 128 that extend in a radial direction along the upward facing surface of barb 112. Projections 120, 122 and channels 128 are constructed and oriented to perforate seal 108 and overcome the plastic deformation of seal 108 in response the only axial translation of container 54 relative to carriage 52. Upon perforation of seal 108 in response to the axial translation of container 54 relative to carriage 52, media 104 egresses or exits volume or cavity 102 of container 54, populates basin 114, interacts with sleeve 90 when employed, and is exposed to the ambient air flows associated with openings 84 defined by carriage 52 during use of dispenser assembly 50. Preferably, one or more optional ribs or standoffs 130 are defined by carriage 52 and oriented about barb 112 proximate the bottom of basin 114. Standoffs 130 maintain a spaced relationship between the distal end of container 54 and the bottom of basin 114 so as to further mitigate or prevent any unintentional sealed interaction therebetween and such that the contents of container 54 can populate basin 114 when available.

After initial deployment, and upon subsequent consumption or evaporation of the volume of media 104 associated with a discrete container 54 such that the desired level of air treatment is no longer provided, service personnel can grip handle 48 of dispenser assembly 50 such that imparting a downward longitudinally directed force disengages tangs 57 of carriage 52 from detents 44 associated with bore 40 of support 38 and disengages dispenser assembly 50 from housing 22. Continued downward longitudinal translation of dispenser assembly 50 effectuates removal of dispenser assembly 50 from housing 22 such that a spent container 54 can be removed from carriage, replaced with a subsequent full container 54, and dispenser assembly 50 can be re-associated with housing 22 of system 20 for continued operation.

Removal of the dispenser assembly 50 from housing 22 allows service personnel to grip upper end 74 of container 54 while grasping handle 48 associated with lower end of carriage 52 to effectuate extrication or removal of the spent or consumed container 54 from sleeve 90, when employed, and carriage 52 during replacement or replenishment of the air treatment media or reconditioning of the discrete dispenser assemblies 50. It is further appreciated that empty or previously deployed containers 54 can be any of recycled, repurposed with other treatment media, or simply discarded. It is further appreciated that, when reconditioned or otherwise having a new or full container 54 associate with carriage 52, user translation of container 54 toward carriage 52 and the dispenser orientation 67 as shown in Fig. 9 can be effectuated by the user while the dispenser assembly 50 is removed from housing 22.

Alternatively, it is appreciated that the recently replaced container 54 be associated with carriage 52 and maintained in the unopened or sealed configuration until such time as the dispenser assembly 50 is introduced to housing 22. That is, it is envisioned that introduction of dispenser assembly 50 to housing 22 effectuate the translation of container 54 relative to carriage 52 between the first and second orientations 65, 67 shown in Figs. 8 and 9, respectively, as tangs 57 approach engagement with detents 44 associated with support 38. That is, it is envisioned that the interior facing surface of cover 24 or base 26 can be constructed to interact with container 54 such that upward translation of carriage 52 relative to housing 22 when a full container 54 is associated therewith effectuates the necessary translation of container 54 relative to carriage 52 to effectuate perforation of the seal 108 associated therewith.

Regardless of the methodology employed to effectuate opening of the discrete containers, volume or cavity 102 of container 54 associated with media 104 is preferably sized to provide suitable air treatment conditioning for a period of at least 30 days and more preferably 60 or more days from introduction of a full container 54 until consumption of the media 104 associated therewith.

Although specific embodiments of the media delivery system 20 and dispenser assembly 50 are illustrated in the drawings and discussed above, it is understood that the size and shape of the media delivery system 20 and dispenser assembly 50 may vary to accommodate various volumes of the cavity 102 within the container 54 of the respective media dispensing system 20. That is to say that the media dispensing or delivery system 20 and dispenser assembly 50 described above and shown in Figs. 1-12 may be provided with a volume or cavity that is sufficient to retain and dispense various volumes of treatment media, all of which are considered will within the scope of the present invention.

Further, the invention may be implemented in a variety of configurations, using certain features or aspects of the embodiments described herein and others known in the art. Thus, although the invention has been herein shown and described in what is perceived to be the most practical and preferred embodiments, it is to be understood that the invention is not intended to be limited to the specific features and embodiments set forth above. Rather, it is recognized that modifications may be made by one of skill in the art of the invention without departing from the spirit or intent of the invention and, therefore, the invention is to be taken as including all reasonable equivalents to the subject matter of the appending claims.

## Claims

1. A passive fragrance dispenser assembly comprising:
a carriage that extends along a longitudinal axis;
a cartridge that defines a cavity configured to contain an amount of an air treatment media and which slideably cooperates with the carriage; and
a barb defined by the carriage and oriented to pierce a seal associated with the cartridge to allow egress of the air treatment media from the cartridge in response to axial translation of the cartridge relative to the carriage and the barb.

2. The passive fragrance dispenser assembly of claim 1 wherein the barb includes a plurality of ridges and a plurality of channels that extend in respective radial directions relative to the plurality of ridges.

3. The passive fragrance dispenser assembly of claim 1 or of claim 2 further comprising a basin defined by the carriage and oriented such that the barb is disposed in the basin.

4. The passive fragrance dispenser assembly of claim 1 or of claim 2 or of claim 3 further comprising a housing configured to support the carriage and the cartridge.

5. The passive fragrance dispenser assembly of claim 4 wherein the housing includes a cover and a base that are movably connected to one another to allow selective exposure of the carriage and cartridge, and optionally or preferably wherein the barb has a conical shape that is aligned with a longitudinal axis of the cartridge.

6. The passive fragrance dispenser assembly of claim 1 or of any preceding claim further comprising at least one tang associated with the carriage and at least one recess associated with the cartridge and configured to receive the at least one tang when the cartridge is engaged with the carriage.

7. An air treatment media dispenser assembly comprising:
a container defined by a body and an opening;
a seal disposed over the opening;
a carriage constructed to slideably receive the container when the seal is oriented in a downward facing direction and secure the container relative to the carriage without interference with the seal; and
a projection defined by the carriage and configured to engage and pierce the seal to fluidly connect a volume defined by the container to atmosphere during axial translation of the container toward the carriage in an axial direction.

8. The air treatment media dispenser assembly of claim 7 wherein the projection includes a plurality of ridges and a plurality of grooves and wherein at least one of the plurality of ridges is oriented in a circumferential direction and at least one of the plurality of grooves extends in a radial direction relative to a longitudinal axis of the proj ection.

9. The air treatment media dispenser assembly of claim 7 or of claim 8 further comprising at least one tang defined by the carriage and constructed to cooperate with the container to secure the container relative to the carriage in at least two discrete axial positions relative to one another, and optionally or preferably further comprising a first indexer defined by the container and a second indexer defined by the carriage wherein the first and second indexers cooperate with one another to prevent rotation between the first indexer and the second indexer when the container and the carriage are associated with one another.

10. The air treatment media dispenser assembly of claim 7 or of claim 8 or of claim 9 further comprising a sleeve disposed between the container and the carriage, and optionally or preferably wherein the sleeve is constructed of a material configured to transport an air treatment media via capillary action.

11. The air treatment media dispenser assembly of claim 7 or of any of claims 8 to 10 wherein the container and the carriage are axially translatable and non-rotatable relative to one another.

12. A method of dispensing an air treatment media to an environment, the method comprising:
associating a sealed cartridge that contains an air treatment media with a carriage; and
translating the sealed cartridge in a downward non-rotational axial direction relative to the carriage to puncture a seal supported by cartridge so that the air treatment media can populate a basin defined by the carriage.

13. The method of claim 12 further comprising securing the sealed cartridge relative to the carriage in a first orientation wherein the seal remains intact and a second orientation wherein the seal is punctured.

14. The method of claim 12 or of claim 13 further comprising disposing a sleeve between the carriage and the sealed cartridge, and optionally or preferably further comprising constructing the sleeve of a capillary material.

15. The method of claim 12 or of claim 13 or of claim 14 further comprising disposing the sealed cartridge and carriage in a perforated housing that is exposed to ambient environment air flows, and optionally or preferably further comprising removing a previously consumed sealed cartridge from the carriage and associating another sealed cartridge with the carriage upon removal of the carriage from the perforated housing.
